# FASCICULE DE BREVET EUROPEEN

(11) **EP 0 684 819 B1**
(45) Date de publication et mention de la délivrance du brevet: **28.11.2001**
(21) Numéro de dépôt: 95904561.8
(22) Date de dépôt: 19.12.1994
(51) Int. Cl.: A61K 31/192, A61K 9/00

(54) **COMPOSITIONS PHARMACEUTIQUES LIQUIDES A BASE D'IBUPROFENE**
FLUESSIGE ARZNEIZUSAMMENSETZUNGEN ENTHALTEND IBUPROFEN
LIQUID PHARMACEUTICAL COMPOSITIONS BASED ON IBUPROFEN

(30) Priorité: 20.12.1993 FR 9315317
(43) Date de publication de la demande: 06.12.1995
(73) Titulaire: Paris, Laurence, 03600 Commentry (FR)
(72) Inventeur: PARIS, Laurence, 03600 Commentry (FR); SINTUREL, Christophe, 03600 Commentry (FR)
(74) Mandataire: Richebourg, Michel François
(86) Numéro de dépôt international: FR9401481
(87) Numéro de publication internationale: WO9517177

(56) Documents cités:
- EP-A- 0 390 369
- EP-A- 0 405 930
- EP-A- 0 468 232
- WO-A-93/13770
- FR-A- 2 400 363
- DATABASE WPI Week 9312, Derwent Publications Ltd., London, GB; AN 93-100632 (12) & WO,A,93 04670 (SHOWA YAKUHIN KAKO KK) 18 Mars 1993

## Description

La présente invention se rapporte au domaine de la pharmacotechnie, c'est-à-dire au domaine de la réalisation de formes galéniques nouvelles, incorporant un principe actif de médicament déjà connu.

La présente invention se rapporte plus particulièrement à une composition pharmaceutique liquide renfermant un principe actif antalgique et/ou anti-inflammatoire en suspension ou en dispersion dans un véhicule visqueux.

La présente invention a spécifiquement pour objet, de nouvelles compositions pharmaceutiques liquides renfermant, à titre de principe actif, de l'ibuprofène en dispersion dans un véhicule aqueux visqueux dont le pH est ajusté.

Le problème posé par la mise en solution de l'Ibuprofène dans un milieu aqueux n'est pas simple à résoudre. En effet, il est possible de salifier l'ibuprofène en le dispersant dans un milieu alcalin où il se dissout. Cependant, la saveur de telles préparations est particulièrement désagréable et les solutions techniques pour dissimuler la saveur amère et brûlante des sels d'Ibuprofène n'ont pas apporté de progrès sensible (voir EP-A-0 351 353 AESCULAPIUS). En effet, même s'il est possible d'enrober l'Ibuprofène et ainsi de dissimuler la saveur immédiate, l'amertume reste et persiste dans la bouche dans les minutes qui suivent l'ingestion.

Il était donc souhaitable de mettre au point une formulation dans laquelle l'Ibuprofène était dispersée d'une manière homogène et stable, tout en assurant la possibilité d'obtenir une saveur et un arôme qui permettent l'utilisation prolongée d'un tel médicament sans appréhension et sans dégoût.

Ces caractéristiques sont nécessaires pour être assuré que le malade suivra le traitement prescrit pendant toute la durée prévue par le médecin. En effet, lorsqu'une composition pharmaceutique présente une saveur peu agréable, le malade est très peu tenté de l'utiliser d'une manière prolongée et, bien souvent, la durée du traitement est écourtée sans bénéfice thérapeutique réel.

Les compositions pharmaceutiques selon l'invention apportent une solution satisfaisante à ce problème en ce que l'lbuprofène est maintenu sous forme insoluble dans le véhicule aqueux, en ce que le véhicule aqueux se présente sous forme visqueuse et même gélifiée et, en ce que l'ensemble est additionné d'un agent de sapidité, d'un agent d'aromatisation, d'un agent édulcorant et d'un agent tampon.

Le véhicule aqueux est une solution ou une dispersion d'un composé polymérique naturel, formant un gel, dérivé de carrhagénines. La caractéristique d'un tel agent gélifiant est de gonfler au contact de l'eau ou sous l'effet d'un agent tensioactif dispersé dans la phase aqueuse.

D'une manière générale, on incorpore à la phase aqueuse de 0,5 à 5 % d'agent gélifiant et tout particulièrement de 0,5 à 2 %.

Pour les besoins de la mise en suspension, il est nécessaire d'utiliser une matière première présentant une granulométrie particulière. C'est ainsi qu'on utilise de l'Ibuproféne très finement pulvérisé dont la majeure partie des grains présente une taille de l'ordre de 10 microns. Cependant, il n'est pas nécessaire d'avoir recours à une qualité micronisée et on peut considérer que la granulométrie la plus appropriée se situe entre 15 et 35 microns. La densité apparente du produit varie de 0,2 à 0,4 g/cm3. La densité après tassage varie de 0.4 à 0,6 g/cm3

Le milieu aqueux est constitué par une solution tampon acide. Le pH de ce milieu joue un rôle très important car il contribue d'une part au maintien à l'état solide de l'lbuprofène en milieu aqueux et d'autre part il est responsable de 1' atténuation de la saveur amère et brûlante du principe actif au contact de la muqueuse buccale. En effet, la muqueuse buccale présente un pH basique qui provoque la solubilisation partielle de l'lbuprofène à son contact et entraîne ainsi, le développement d'une saveur désagréable, amère et brûlante. Par action de la solution tampon, à son niveau, cet effet est supprimé grâce à sa capacité de compenser l'alcalinité du milieu buccal tout en maintenant un pH acide. A un degré moindre, le pH du milieu contribue à procurer aux compositions liquides, selon l'invention, une saveur qui soit compatible avec l'effet édulcorant et/ou aromatisant des autres additifs. A cet égard, on a trouvé qu'un pH compris entre 1 et 5 et plus particulièrement entre 3 et 4 et surtout entre 3,2 et 3,8 convenait bien et contribuait à donner une saveur de fruits agréable

Cette zone de pH est obtenue en ajoutant à la préparation un acide organique relativement faible comme l'acide citrique ou l'acide tartrique et le milieu est tamponné à cette valeur de pH par addition d'une petite quantité d'un agent alcalin comme par exemple le phosphate disodique ou le citrate monosodique. On peut utiliser également d'autres agents tampon comme l'acide phosphorique, le mono phosphate de sodium, l'acide lactique ou l'acide chlorhydrique.

L'agent de sapidité est en règle générale un polyol. Un agent particulier est choisi notamment parmi le xylitol, le mannitol, le sorbitol, le lactitol, I'iditol ou le glucitol. Le sorbitol ou encore le glycérol sont les agents de sapidité préférés. Leur concentration dans les compositions selon l'invention s'échelonne de 10 à 50 g% de la masse totale et de préférence de 20 à 40 g%.

Les compositions selon l'invention renferment en outre un agent mouillant destiné à assurer une bonne dispersion du principe actif. L'agent mouillant est un agent tensioactif non ionique comme un Tween®, un Span® , un Brij® ou un copolymère en bloc polyéthylène/polypropylène. On peut également utiliser un agent tensioactif anionique comme un lauryl sulfate de sodium à des concentrations faibles (0,1 à 0,5 %).

La quantité d'agent mouillant non ionique ajouté à la composition s'échelonne entre 0,1 et 1 % en poids de la préparation et de préférence de 0,2 à 0,5 g%.

L'agent édulcorant est choisi dans le groupe des édulcorants de synthèse autorisés pour l'usage pharmaceutique. On citera à cet égard la saccharine et ses sels, l'aspartame, l'acésulfame, le cyclamate de sodium et les mélanges de ces différents composés. La quantité d'agent édulcorant ajouté dépend de son pouvoir sucrant. Il correspond à une concentration de 0,005 à 1% selon les cas.

La substance aromatisante ajoutée à la composition est un arôme de fruits et l'arôme de fraise, naturel ou synthétique, est celui qui a fourni l'aromatisation la plus appropriée.

L'invention concerne également un procédé de réalisation des compositions liquides à base d'Ibuprofène qui consiste à dissoudre sous agitation l'agent d'ajustement du pH dans un grand volume d'eau puis à y ajouter l'agent mouillant et l'agent de sapidité. On introduit alors l'lbuprofène en fine dispersion et on maintient l'agitation jusqu'à obtenir une suspension homogène.

Dans un autre récipient, on introduit l'agent gélifiant dans un petit volume d'eau sous forte agitation, à une température comprise entre 10 et 20°C. On maintient l'agitation jusqu'à obtention d'une masse visqueuse homogène, on laisse reposer plusieurs heures pour permettre au milieu de développer une viscosité maximale. A ce gel est ajoutée la suspension tamponnée d'Ibuprofène, ceci sous grande vitesse. A cet ensemble, on ajoute l'agent de conservation, l'agent d'édulcoration, l'agent d'aromatisation et, s'il y a lieu, l'agent colorant.

On maintient sous agitation la suspension pendant toute la durée de l'addition.

La concentration en principe actif peut varier de 1 à 10% en poids de façon à pouvoir réaliser une suspension contenant de 25 à 200 mg, et de préférence de 100 mg ou 200 mg d'Ibuprofène dans un volume de liquide s'échelonnant de 1 à 10 ml et de préférence, une suspension contenant 100 mg d'Ibuprofène dans 5 ml de solution.

La suspension peut contenir, en outre, un autre principe actif d'action similaire ou complémentaire ou synergique et notamment, un antalgique comme la codéine ou le dextropropoxyphène.

Les compositions selon l'invention trouvent un emploi en thérapeutique comme médicament antalgique et à des doses plus élevées, comme anti-inflammatoire.

L'exemple de réalisation figurant ci-après est un des modes de réalisation possibles des compositions selon l'invention et ne la limite en aucune façon.

## Revendications

1. Compositions pharmaceutiques liquides à base d'Ibuprofène **caractérisées en ce que** l'ibuprofène est sous forme dispersée dans une solution aqueuse d'un composé polymérique naturel choisi parmi les carrhagénines formant un gel dont le pH est ajusté entre 1 et 5, et **en ce qu'**elles renferment un agent mouillant choisi parmi les agents tensio-actifs non-ioniques et les agents tensio-actifs anioniques.

2. Composition pharmaceutique selon la revendication 1 dans laquelle la valeur du pH est ajustée entre 3,0 et 4,0.

3. Composition pharmaceutique selon la revendication 2 dans laquelle la valeur du pH est ajustée au moyen d'une solution tampon acide.

4. Composition pharmaceutique selon les revendications 1 à 3 prises ensemble dans laquelle l'agent mouillant est un agent tensio-actif non ionique.

5. Composition pharmaceutique selon l'une des revendications 1 à 4 dans laquelle l'agent de sapidité est un polyol choisi parmi les triols, les hexitols, les pentitols et les dihexitols.

6. Composition pharmaceutique selon l'une des revendications 1 à 5 dans laquelle la dispersion aqueuse contient en outre un agent édulcorant choisi dans le groupe formé de la saccharine, de l'aspartame, de l'acesulfame et du cyclohexyl sulfamate de sodium.

7. Composition pharmaceutique selon l'une des revendications 1 à 6 dans laquelle on ajoute aussi un agent d'aromatisation.

8. Composition pharmaceutique selon l'une des revendications 1 à 7 dans laquelle l'agent d'aromatisation est un arôme de fruits.

9. Composition pharmaceutique selon l'une des revendications précédentes dans laquelle la teneur en principe actif s'échelonne de 500 mg à 2500 mg pour 100 ml.

10. Composition pharmaceutique selon la revendication 9 dans laquelle on ajoute au principe actif un autre médicament antalgique d'action similaire, complémentaire ou synergique.

11. Procédé de production de compositions pharmaceutiques liquides à base d'ibuprofène selon la revendication 1 qui consiste à réaliser dans un premier temps, une solution aqueuse d'un agent de sapidité additionnée d'un agent tampon permettant d'ajuster le pH, et d'un agent mouillant, d'y disperser l'ibuprofène en poudre d'une granulométrie déterminée, à réaliser à part une dispersion d'un agent gonflant qu'on laisse reposer jusqu'à formation d'un gel, à ajouter la suspension de principe actif sous agitation, à la solution d'agent gonflant et à maintenir l'agitation jusqu'à obtention d'une dispersion aqueuse visqueuse, homogène puis à y ajouter un agent édulcorant et un agent d'aromatisation et les adjuvants usuels et finalement, à ajuster la suspension à la concentration désirée par addition d'eau.

## Patentansprüche

1. Flüssige pharmazeutische Zusammensetzungen auf Ibuprofenbasis, **dadurch gekennzeichnet, daß** das Ibuprofen in dispergierter Form in einer wässrigen Lösung mit einer natürlich vorkommenden Polymerverbindung aus der Gruppe der Karrhagene vorliegt, wobeil der pH-Wert zwischen 1 und 5 eingestellt wird, und daß sie ein Netzmittel aus der Gruppe der nichtionogenen Tenside oder Anionentenside enthalten.

2. Pharmazeutische Zusammensetzung nach Anspruch 1, bei der der pH-Wert auf 3,0 bis 4,0 eingestellt ist.

3. Pharmazeutische Zusammensetzung nach Anspruch 2, bei der der pH-Wert mittels einer saueren Pufferlösung eingestellt wird.

4. Pharmazeutische Zusammensetzung gemäß den zusammengenommenen Patentansprüchen 1 bis 3, bei der das Netzmittel ein nichtionogenes Tensid ist.

5. Pharmazeutische Zusammensetzung nach einem der Ansprüche 1 bis 4, bei der der Geschmacksstoff ein mehrwertiger Alkohol ist, der aus der Gruppe der Triolen, Hexitolen, Pentitolen oder Dihexitolen gewählt wird.

6. Pharmazeutische Zusammensetzung nach einem der Ansprüche 1 bis 5, bei der die wässrige Dispersion außerdem einen Süßstoff enthält, der unter Saccharin, Aspartam, Acesulfam und Cyclamat gewählt wird.

7. Pharmazeutische Zusammensetzung nach einem der Ansprüche 1 bis 6, bei der auch ein Aromastoff hinzugefügt wird.

8. Pharmazeutische Zusammensetzung nach einem der Ansprüche 1 bis 7, bei der der Aromastoff ein Fruchtaroma ist.

9. Pharmazeutische Zusammensetzung nach einem der vorstehenden Ansprüche, bei dem der Wirkstoffgehalt bei 500-2500 mg/100 ml liegt.

10. Pharmazeutische Zusammensetzung nach Anspruch 9, bei dem zum Wirkstoff ein weiteres Analgetikum mit ähnlicher, komplementärer oder synergistischer Wirkung hinzugefügt wird.

11. Verfahren zur Herstellung von flüssigen pharmazeutischen Zusammensetzungen auf Ibuprofenbasis nach Anspruch 1, das darin besteht, daß zunächst eine wässrige Lösung mit einem Geschmackswirkstoff hergestellt wird, der ein Puffer zur Einstellung vom pH-Wert und ein Netzmittel zugefügt werden, in dem das pulverförmige Ibuprofen einer bestimmten Korngröße dispergiert wird, wobei separat eine Dispersion mit einem Quellmittel hergestellt wird, die man ruhen lässt, bis sich ein Gel gebildet hat, wonach die Suspension mit dem Wirkstoff der Quellmittellösung unter Rühren hinzugefügt wird und so lange weitergerührt wird, bis eine wässrige, visköse, homogene Dispersion entsteht, wonach ein Süßstoff und ein Aromastoff sowie die üblichen Hilfsstoffe zugesetzt werden und die Suspension schließlich durch Zugabe von Wasser auf die gewünschte Konzentration eingestellt wird.

## Claims

1. Liquid pharmaceutical compound with an Ibuprofen basis, wherein ibuprofen is under a form dispersed in an aqueous solution of a natural polymeric compound chosen among the carrageenans forming a gel, the pH of which is adjusted between 1 and 5, and where said carrageenans contain a wetting agent chosen among the surface-active non ionic agents and the anionic surface-active agents.

2. Pharmaceutical compound according to claim 1, wherein the value of the pH is adjusted between 3.0 and 4.0.

3. Pharmaceutical compound according to claim 2, wherein the value of the pH is adjusted by means of an acid buffer solution.

4. Pharmaceutical compound according to claims 1 to 3 taken together, wherein the wetting agent is a surface-active non ionic agent.

5. Pharmaceutical compound according to any one of claims 1 to 4, wherein the sapidity agent is a polyalcohol chosen among triols, hexitols, pentitols, and dihexitols.

6. Pharmaceutical compound according to any one of claims 1 to 5, wherein the aqueous dispersion moreover contains a sweetener chosen in the group formed by saccharin, aspartame, acesulfame, and sodium cyclohexyl- sulfamate.

7. Pharmaceutical compound according to any one of claims 1 to 6, wherein a flavoring agent is also added.

8. Pharmaceutical compound according to any one of claims 1 to 7, wherein the flavoring agent is a fruit aroma.

9. Pharmaceutical compound according to any one of the preceding claims, wherein the content of the active principle is comprised between 500 mg and 2500 mg per 100 ml.

10. Pharmaceutical compound according to claim 9, wherein to the active principle is added another antalgic medication having a similar, complementary or synergetic action.

11. Method for manufacturing liquid pharmaceutical compounds with an ibuprofen basis according to claim 1, which consists in obtaining in a first stage an aqueous solution of a sapidity agent in which are added a buffer agent allowing pH adjustment and a wetting agent, in dispersing in said solution the ibuprofen under powder form having a set granulometry, in obtaining in another container a dispersion of a swelling agent which is laid to rest until a gel is formed, in adding the suspension of active principle while shaking to the solution of the swelling agent and in maintaining the shaking until an homogeneous, viscous and liquid dispersion is obtained, then in adding to said dispersion a sweetener and an flavoring agent as well as the usual additives, and finally in adjusting the suspension at the desired concentration by adding water.
